(19) Europäisches Patentamt / European Patent Office / Office européen des brevets

(11) **EP 3 381 367 B1**

(12) **FASCICULE DE BREVET EUROPEEN**

(45) Date de publication et mention
de la délivrance du brevet:
**02.02.2022   Bulletin 2022/05**

(51) Classification Internationale des Brevets (IPC):
**G01C 25/00** (2006.01)     **G01C 21/16** (2006.01)
**A61B 5/11** (2006.01)     **A61B 5/00** (2006.01)

(21) Numéro de dépôt: **18173359.3**

(22) Date de dépôt: **21.04.2008**

(52) Classification Coopérative des Brevets (CPC):
**A61B 5/1126; G01C 21/16; G01C 25/00;**
A61B 5/6824; A61B 2560/0223; A61B 2562/0219

(54) **PROCÉDÉ ET DISPOSITIF DE CALIBRATION D'UN CAPTEUR INERTIEL OU MAGNÉTIQUE À TROIS AXES DE SENSIBILITÉ**

VERFAHREN UND VORRICHTUNG ZUM KALIBRIEREN EINES TRÄGHEITS- ODER MAGNETSENSORS MIT DREI SENSIBILITÄTSACHSEN

METHOD AND DEVICE FOR CALIBRATING A MAGNETIC OR INERTIAL SENSOR, WITH THREE AXES OF SENSITIVITY

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **25.04.2007   FR 0754693**

(43) Date de publication de la demande:
**03.10.2018   Bulletin 2018/40**

(62) Numéro(s) de document de la (des) demande(s)
initiale(s) en application de l'article 76 CBE:
**08290390.7 / 1 985 233**

(73) Titulaire: **Commissariat à l'Énergie Atomique et aux Énergies Alternatives 75015 Paris (FR)**

(72) Inventeurs:
• **GODIN, Christelle**
  **38054 Grenoble Cedex 09 (FR)**
• **BONNET, Stéphane**
  **38054 Grenoble Cedex 09 (FR)**
• **BARRAUD, Alain**
  **38190 Froges (FR)**
• **LESECQ, Suzanne**
  **38054 Grenoble Cedex 09 (FR)**

(74) Mandataire: **Santarelli**
  **49, avenue des Champs-Elysées**
  **75008 Paris (FR)**

(56) Documents cités:
**US-A- 3 731 521          US-A- 5 105 152
US-A1- 2002 103 610**

• **CHUNLEI RUI ET AL: "Nonlinear Attitude and Shape Control of Spacecraft with Articulated Appendages and Reaction Wheels", IEEE TRANSACTIONS ON AUTOMATIC CONTROL, IEEE SERVICE CENTER, LOS ALAMITOS, CA, US, vol. 45, no. 8, août 2000 (2000-08), XP011001383, ISSN: 0018-9286**

**Description**

La présente invention concerne la calibration d'un capteur inertiel ou magnétique à trois axes de sensibilité.

De manière générale, la présente invention concerne le domaine de la capture de mouvements. Les domaines d'application sont variés et couvrent en particulier le domaine biomédical, pour l'observation des personnes, le domaine sportif, pour l'analyse d'un mouvement, d'un sportif lui-même ou de son équipement (raquettes, balles ...). Elle s'applique également au domaine de l'automobile ou de la robotique, ainsi qu'à la réalité virtuelle, et de manière générale à toute application comportant un corps mobile dont on cherche à déterminer ou à observer le mouvement.

On connaît en particulier des systèmes inertiels constitués d'un ou plusieurs capteurs choisis notamment parmi des accéléromètres, des magnétomètres ou des gyromètres, ayant un ou plusieurs axes de sensibilité.

Ces systèmes inertiels présentent l'avantage d'être autonomes et de ne pas requérir d'équipement préalable de l'environnement dans lequel on observe le mouvement d'un mobile.

On connaît en particulier un dispositif de capture de mouvement de rotation d'un solide tel que décrit dans le document FR 2 838 185. Le document US2002/103610 divulgue un procédé de calibration et un procédé de détermination de l'orientation d'un capteur, comportant la mesure d'un vecteur de champ magnétique et d'un vecteur de gravité.

Lors de l'observation d'un mobile en mouvement, le principe consiste à rechercher les grandeurs représentatives du déplacement et de l'orientation du mobile, ou encore de sa vitesse et vitesse de rotation ou de son accélération et accélération angulaire.

En pratique, les capteurs sont disposés sur le mobile observé et on cherche à déterminer un mouvement ayant potentiellement six degrés de liberté, c'est-à-dire trois degrés de liberté correspondant à l'orientation du solide dans l'espace et trois degrés de liberté correspondant à la position du mobile.

La résolution des systèmes d'équations pour rechercher les grandeurs représentatives du mouvement d'un solide est généralement complexe et nécessite des systèmes de résolution dans lesquels certaines contraintes sont connues par avance.

La présente invention a pour but de résoudre les inconvénients des systèmes d'estimation de mouvement antérieurs pour la calibration d'un capteur inertiel ou magnétique et de proposer un procédé particulièrement bien adapté au traitement du mouvement d'un mobile ayant un axe de rotation sensiblement invariant dans le cadre d'un procédé de calibration d'un capteur inertiel ou magnétique à trois axes de sensibilité.

Selon un premier aspect, il est décrit un procédé de détection d'un axe de rotation sensiblement invariant d'un mouvement d'un mobile équipé d'au moins un capteur inertiel ou magnétique à trois axes de sensibilité.

Ce procédé de détection comprend les étapes suivantes :

- acquisition de mesures physiques selon les trois axes de sensibilité du capteur ;
- estimation d'un axe de rotation sensiblement invariant dans l'espace des mesures physiques ; et
- identification dudit axe estimé comme axe de rotation sensiblement invariant du mouvement.

En effet, la demanderesse a constaté qu'en déterminant l'axe de rotation sensiblement invariant parmi les mesures obtenues en sortie d'un même capteur, il est possible de déterminer par là même l'axe de rotation sensiblement constant du mouvement du mobile équipé de ce capteur.

En pratique, le procédé de détection comprend en outre les étapes suivantes :

- calcul d'un indicateur des variations de l'axe de rotation ;
- comparaison de la valeur de l'indicateur avec une valeur de seuil prédéterminée ; et
- validation de l'axe de rotation estimé comme axe de rotation sensiblement invariant du mouvement lorsque la valeur de l'indicateur est inférieure à la valeur de seuil prédéterminée, avantageusement égale à la valeur du bruit des mesures physiques dudit capteur.

Ainsi, grâce à un indicateur de variations de l'axe de rotation estimé, il est possible d'obtenir une indication sur les variations de l'axe de rotation du mouvement. Si la valeur de cet indicateur est suffisamment faible, c'est-à-dire de l'ordre de la valeur du bruit des mesures physiques du capteur observé, on peut considérer que le mouvement correspond bien à une rotation autour d'un axe sensiblement invariant. Il est ainsi possible de valider l'axe de rotation estimé dans l'espace des mesures physiques comme axe de rotation sensiblement invariant du mouvement du mobile.

Avantageusement, les mesures physiques comprennent N échantillons à des instants différents des mesures physiques selon les trois axes de sensibilité dudit capteur, N étant supérieur ou égal 3.

En effet, l'estimation de l'axe de rotation dans l'espace des mesures physiques nécessite de disposer d'au moins trois mesures différentes à des instants différents et selon les trois axes de sensibilité du capteur, pour permettre la détermination des coordonnées de l'axe de rotation estimé.

Selon un mode de réalisation pratique, lorsque le mobile est équipé d'au moins un magnétomètre à trois axes de sensibilité et d'un accéléromètre à trois axes de sensibilité, le procédé de détection comprend les étapes suivantes :

- acquisition des mesures physiques selon les trois axes de sensibilité respectivement du magnétomètre et de l'accéléromètre ;
- estimation des axes de rotation sensiblement invariants dans les espaces desdits mesures physiques respectivement dudit magnétomètre et dudit accéléromètre ; et
- validation, comme axe de rotation sensiblement invariant dudit mouvement, d'une combinaison linéaire des axes de rotation estimés pour lesdites mesures physiques du magnétomètre et de l'accéléromètre, les coefficients de la combinaison étant compris non strictement entre 0 et 1, leur somme étant égale à 1 et étant fonction des valeurs des indicateurs des variations des axes de rotation du magnétomètre et/ou de l'accéléromètre.

En particulier, lorsque l'un des coefficients de la combinaison est égal à 0, il est possible d'utiliser l'un ou l'autre capteur (magnétomètre ou accéléromètre) permettant d'obtenir l'estimation la plus fiable de l'axe de rotation sensiblement invariant.

En particulier, lorsque la rotation a lieu autour d'un axe du champ physique considéré (le champ de gravité pour les accéléromètres, le champ magnétique terrestre pour les magnétomètres), il est possible de déterminer l'axe de rotation invariant en utilisant l'autre capteur, dès lors que l'indicateur des variations de l'axe de rotation estimé au moyen des mesures physiques du capteur associé au champ physique invariant sera nécessairement mauvais, c'est-à-dire très supérieur à la valeur du bruit des mesures physiques obtenues par ce capteur.

Dans un mode de réalisation pratique, le procédé de détection comporte en outre une étape de transformation des coordonnées de l'axe de rotation estimé dans le repère de mesure du capteur en coordonnées dans un repère géophysique, tel que dans un repère NED (acronyme pour *North-East-Down).*

Selon un second aspect, il est décrit un procédé d'estimation d'un mouvement d'un mobile équipé d'au moins un capteur inertiel ou magnétique à trois axes de sensibilité, le mouvement comprenant une rotation autour d'un axe sensiblement invariant.

Le procédé d'estimation comprend les étapes suivantes :

- détection d'un axe de rotation sensiblement invariant selon le procédé de détection décrit ; et
- détermination de l'angle de rotation du mobile autour de l'axe de rotation sensiblement invariant.

Lorsque le capteur est un magnétomètre ou un goniomètre et que le mobile est équipé en outre d'un accéléromètre, le procédé d'estimation comprend en outre une étape de détermination de l'accélération du mobile.

Grâce au procédé décrit de détection d'un axe de rotation sensiblement invariant, le procédé d'estimation du mouvement d'un mobile est facilité dès lors que le nombre d'inconnues à déterminer est limité à la détermination de l'angle de rotation, et éventuellement de l'accélération du mobile.

Après détection de l'axe de rotation invariant conformément au procédé décrit, il est possible de calculer ce mouvement en deux étapes, par la recherche tout d'abord de l'angle de rotation, puis le calcul de l'accélération du mobile.

L'invention concerne un procédé de calibration selon la revendication 1 d'un capteur inertiel ou magnétique à trois axes de sensibilité, permettant de déterminer une matrice de rotation adaptée à transformer les coordonnées exprimées dans un repère de mesure du capteur en des coordonnées exprimées dans un repère prédéterminé solidaire d'un mobile portant le capteur.

Ce procédé de calibration comprend les étapes suivantes :

- acquisition d'au moins deux séries de mesures physiques par le capteur correspondant respectivement à la rotation du mobile selon au moins deux des trois axes du repère prédéterminé solidaire du mobile ;
- détermination à partir de chacune des deux séries des coordonnées de chacun des axes de rotation correspondant à l'un desdits deux des trois axes du repère prédéterminé solidaire du mobile dans le repère des mesures physiques du capteur selon le procédé de détection décrit ; et
- élaboration d'une matrice de rotation à partir des coordonnées déterminées desdits au moins deux des trois axes de rotation.

Ce procédé de calibration permet ensuite d'exprimer les mesures obtenues au niveau du capteur dans un repère prédéterminé lié au mobile observé, tel qu'un boîtier renfermant le capteur ou encore une partie anatomique d'un individu sur laquelle est positionné le capteur.

Enfin, il est décrit un dispositif de détection d'un axe de rotation sensiblement invariant d'un mouvement d'un mobile équipé d'au moins un capteur inertiel ou magnétique à trois axes de sensibilité.

Ce dispositif de détection comprend des moyens adaptés à mettre en œuvre le procédé de détection décrit.

Ce dispositif de détection présente des caractéristiques et avantages analogues à ceux décrits précédemment en relation avec le procédé de détection.

D'autres particularités et avantages de l'invention apparaîtront dans la description ci-après.

Aux dessins annexés, donnés à titre d'exemple non limitatif :

- la figure 1 est un algorithme illustrant un procédé de détection d'un axe de rotation sensiblement invariant conforme à un premier mode de réalisation ;
- la figure 2 est un schéma illustrant l'étape d'estimation d'un axe de rotation sensiblement invariant ;
- la figure 3 est un algorithme illustrant un procédé de détection d'un axe de rotation sensiblement invariant selon un second mode de réalisation ;
- la figure 4 est un algorithme illustrant un procédé d'estimation d'un mouvement d'un mobile conformément à un mode de réalisation ;
- la figure 5 est un schéma illustrant un dispositif de calibration conforme à un mode de réalisation de l'invention, intégré dans un boîtier ;
- la figure 6 est un schéma illustrant un dispositif de calibration conforme à un mode de réalisation de l'invention, dans lequel le capteur est positionné sur une partie anatomique ; et
- la figure 7 est un algorithme illustrant le procédé de calibration d'un capteur conforme à un mode de réalisation de l'invention.

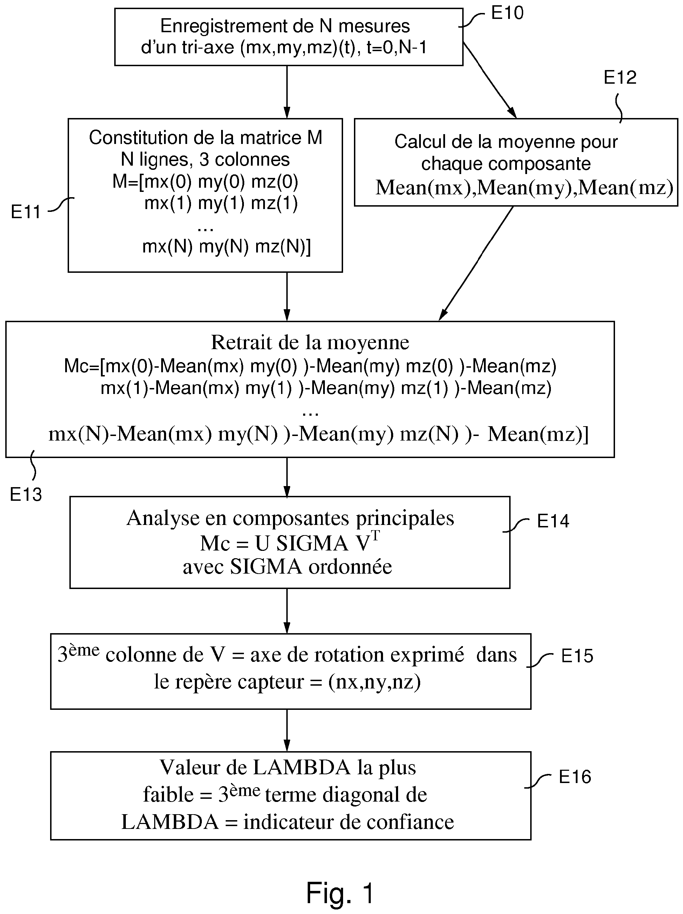

On va décrire tout d'abord en référence à la figure 1, un premier mode de réalisation d'un procédé de détection d'un axe de rotation sensiblement invariant d'un mouvement d'un mobile équipé d'un capteur à trois axes de sensibilité.

Ce procédé peut s'appliquer à l'étude du mouvement d'un mobile dans de nombreuses applications dès lors que le mouvement de ce mobile correspond à une rotation autour d'un axe fixe ou sensiblement invariant.

Il existe en effet de nombreuses applications dans lesquelles le mouvement du mobile observé correspond à une rotation autour d'un axe, avec en outre généralement des déplacements nuls selon cet axe de rotation de telle sorte que le mouvement est réalisé dans un plan et peut être considéré comme un mouvement planaire.

A titre d'exemple, un tel mouvement est observé pour un véhicule dans lequel le roulis et le tangage sont nuls. La rotation du véhicule correspond à une rotation autour d'un axe vertical fixe, correspondant à un mouvement de lacet. Généralement, l'accélération verticale est nulle de telle sorte que le mouvement du véhicule est un mouvement planaire.

De même, l'observation de mouvements des membres d'une personne (bras, jambes, ...) est réalisée dans un plan dès lors qu'il n'existe qu'un seul axe de rotation autour d'une articulation et un angle qui varie au cours du temps.

Le mouvement d'une roue qui roule sans glissement sur un plan et en ligne droite correspond également à un mouvement autour d'un axe de rotation, perpendiculaire au plan de la roue.

Le déplacement d'une souris d'ordinateur dans un plan autour d'un axe orthogonal à ce plan est également un mouvement planaire qui peut être observé dans le cadre de la présente invention.

De manière générale, le procédé de détection de l'axe de rotation sensiblement invariant d'un tel mouvement est mis en œuvre à l'aide d'un dispositif de détection équipé d'au moins un capteur à trois axes de sensibilité, tel que par exemple un accéléromètre, un magnétomètre ou un gyromètre monté en tri-axe.

Dans son principe, le procédé de détection consiste à estimer un axe de rotation sensiblement invariant dans l'espace des mesures physiques obtenues par le capteur à trois axes de sensibilité.

Dans le mode de réalisation illustré à la figure 1, on considère que l'axe de rotation sensiblement invariant dans l'espace des mesures correspond à l'axe de plus faible variation des mesures pour le magnétomètre et l'accéléromètre et de plus grande variation pour le gyromètre.

Chaque mesure à un instant t obtenue en sortie d'un capteur à trois axes de sensibilité est considérée comme un point dans l'espace des mesures à trois dimensions.

L'axe invariant correspond ainsi à l'axe pour lequel les mesures présentent pour un magnétomètre ou un accéléromètre la plus faible variation sur cet axe, c'est-à-dire que l'écart-type des points projetés sur cet axe est le plus faible.

Dans le cas d'un gyromètre, ce sera l'axe de plus grande variation puisque le gyromètre mesure sur chacun de ses axes, la valeur de la rotation autour de cet axe.

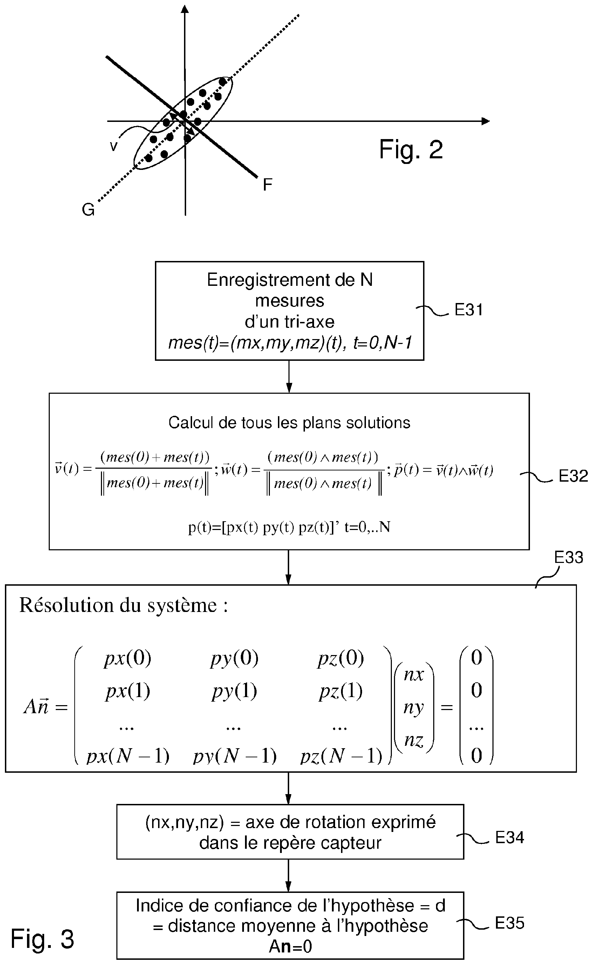

On a illustré à la figure 2, de manière simplifiée dans un espace à deux dimensions, des mesures représentées par un ensemble de points.

Bien entendu, cette représentation schématique peut être étendue à un espace à trois dimensions.

L'axe de plus faible variation F et l'axe de plus grande variation G sont illustrés sur la figure 2.

La flèche v selon la direction de l'axe de plus faible variation F correspond à l'écart type des mesures projetées sur cet axe et correspond ainsi à un indicateur de variations de l'axe de rotation. Plus cette variance v est faible, plus le mouvement observé correspondra à un mouvement de rotation autour d'un axe fixe.

On va décrire en référence à la figure 1 un procédé de détection d'un axe de rotation sensiblement invariant permettant de déterminer l'axe de plus faible variation à partir d'une analyse en composantes principales.

En pratique, une étape d'acquisition E10 de mesures physiques selon les trois axes de sensibilité du capteur est mise en œuvre.

Les mesures physiques comprennent N échantillons à des instants différents selon les trois axes de sensibilité du capteur.

Afin de permettre la résolution du problème, N est supérieur ou égal à 3.

On enregistre ainsi N mesures (mx, my, mz)(t) avec t variant de 0 à N - 1.

Une étape de constitution E11 d'une matrice M est ensuite mise en œuvre de telle sorte que chaque ligne correspond à un échantillon temporel des mesures et que chaque colonne correspond à une composante obtenue selon les axes de sensibilité X, Y, Z du capteur.

On obtient ainsi :

$$M= \begin{matrix} [mx(0) \ my(0) \ mz(0) \\ mx(1) \ my(1) \ mz(1) \\ \dots \\ mx(N) \ my(N) \ mz(N)] \end{matrix}$$

Simultanément, une étape de calcul E12 de la moyenne obtenue pour chaque composante selon les axes de sensibilité X, Y, Z est mise en œuvre.

On calcule ainsi en pratique une moyenne des mesures obtenues sur chaque colonne de la matrice M.

Les moyennes suivantes sont déterminées :
Mean(mx), Mean(my), Mean(mz)

Une étape d'homogénéisation E13 est ensuite mise en œuvre afin de soustraire la valeur moyenne calculée à l'étape E12 de chaque élément de la matrice M. On obtient ainsi une matrice Mc :

$$Mc= \begin{matrix} [mx(0)\text{-}Mean(mx) & my(0)\text{-}Mean(my) & mz(0)\text{-}Mean(mz) \\ mx(1)\text{-}Mean(mx) & my(1)\text{-}Mean(my) & mz(1)\text{-}Mean(mz) \\ \dots \\ mx(N)\text{-}Mean(mx) & my(N)\text{-}Mean(my) & mz(N)\text{-}Mean(mz)] \end{matrix}$$

Sur la matrice obtenue Mc, il est possible de réaliser une analyse en composantes principales dans une étape d'analyse E14.

La détermination de l'axe de plus faible variation par une analyse en composante principale est connue et n'a pas besoin d'être décrite en détail ici. On pourra se reporter notamment à la description de cette technique faire dans le recueil Jolliffe I.T., Principal component analysis, 2nde édition, Springer 2002.

Dans son principe, cette analyse en composantes principales revient à rechercher une matrice unitaire V de dimensions 3 × 3, dans laquelle chaque vecteur colonne est de norme unitaire et orthogonal aux autres vecteurs colonnes, une matrice diagonale ordonnée SIGMA de taille 3 × 3 avec des éléments positifs de la diagonale en ordre décroissant et une matrice unitaire U de dimensions N × N telle que :

$$Mc = U \ SIGMA \ V^T$$

Ces matrices peuvent être obtenues en réalisant la décomposition en valeurs singulières de la matrice Mc ou la décomposition en valeurs propres et vecteurs propres de la matrice S = (N - 1) $Mc^TMc$, de telle manière que S V = V LAMBDA où LAMBDA est une matrice diagonale qui contient les valeurs propres de S. On a de plus LAMBDA (i, i) = $(N-1)^{-1}$ SIGMA $(i, i)^2$.

Les colonnes de la matrice unitaire V sont appelées axes factoriels et l'axe de rotation recherché exprimé dans le repère associé aux capteurs, correspondant à l'axe de projection sur lequel les mesures ont la plus faible variance, correspond à la troisième colonne de la matrice unitaire V.

La valeur LAMBDA (3,3) sur la troisième colonne de la matrice diagonale LAMBDA correspond à la variance associée à l'axe ainsi déterminé.

Il est ainsi possible d'estimer l'axe de rotation sensiblement invariant dans une étape d'estimation E15 à partir de l'axe factoriel de la troisième colonne de la matrice unitaire V, l'axe de rotation étant ainsi exprimé dans le repère associé aux capteurs. Cet axe de rotation sera noté dans la suite par ses coordonnées dans le repère du capteur de la manière suivante :

(nx, ny, nz)

Afin de valider l'axe de rotation ainsi estimé, le procédé de détection comporte en outre une étape de calcul E16 d'un indicateur des variations de l'axe de rotation.

On calcule ainsi la valeur d de telle sorte que :

$$d = \frac{LAMBDA\ (3,3)}{LAMBDA\ (1,1) + LAMBDA\ (2,2) + LAMBDA\ (3,3)}$$

Cette valeur d de l'indicateur donne une indication sur la validation de l'axe de rotation estimé comme axe de rotation invariant du mouvement.

Plus la valeur d est petite, plus l'hypothèse selon laquelle le mouvement du mobile est réalisé autour d'un axe de rotation constant ou sensiblement invariant est valable.

En pratique, on compare la valeur de LAMBDA (3,3) de l'indicateur avec la valeur du bruit des mesures physiques obtenues par le capteur. Lorsque la valeur de LAMBDA (3,3) de l'indicateur est sensiblement égale ou du même ordre de grandeur que la valeur du bruit, l'axe de rotation estimé (nx, ny, nz) est validé comme axe de rotation sensiblement invariant du mouvement du mobile.

D'autre part, il faut que les variations selon les autres axes soient importantes : LAMBDA (3,3) + LAMBDA (2,2) + LAMBDA (1,1) doit être grand ce qui garantit que le mouvement réalisé est de suffisamment grande amplitude.

En pratique, si d est proche de 1/3, les trois axes ont des variations équivalentes et l'on ne peut rien conclure. On ne conservera l'hypothèse "axe de rotation constant" que si d<seuil que l'on prendra égal à 0.1 par exemple.

Un autre indicateur peut être d*= LAMBDA (3,3) / LAMBDA (2,2) qui devra être petit devant 1. On ne conservera l'hypothèse "axe de rotation constant" que si d*<seuil que l'on prendra égal à 0.1 par exemple.

D'autres méthodes permettant d'estimer l'axe de plus faible variation dans les mesures obtenues par un capteur inertiel ou magnétique peuvent également être mises en œuvre.

A titre d'exemple, la méthode des moindres carrés peut également être utilisée.

Dans son principe, la méthode des moindres carrés revient à estimer l'axe n sur lequel la projection des mesures M est constante. Cet axe n peut être calculé par la formule suivante :

$$n = pinv(M)O \text{ où}$$

pinv(M) est l'inverse généralisé de la matrice M telle que constituée à l'étape E11 décrite en relation à la figure 1,
n est un vecteur à trois composantes en colonne $n = (nx, ny, nz)^T$, et
O est un vecteur de N lignes et 1 colonne dont tous les éléments sont égaux à 1.

On normalise le vecteur n pour obtenir le vecteur directeur de la normale au plan.

On notera de manière générale que le procédé de détection d'un axe de rotation décrit précédemment peut être mis en œuvre pour différents types de capteurs inertiels ou magnétiques et par exemple un accéléromètre à trois axes de sensibilité ou un magnétomètre à trois axes de sensibilité ou encore un gyromètre à trois axes de sensibilité.

Toutefois, dans le cas d'un capteur inertiel du type gyromètre, l'axe de rotation estimé correspond non pas à l'axe de plus faible variation, mais au contraire à l'axe de plus grande variation des mesures. L'indicateur des variations de l'axe de rotation utilisé correspond alors à la variance ou l'écart-type, calculé sur les deux axes orthogonaux à l'axe de rotation estimé.

Bien entendu, un seul capteur inertiel ou magnétique permet à partir des mesures physiques en sortie de ce capteur de détecter l'axe de rotation constant sous réserve que la valeur de l'indicateur des variations de l'axe de rotation soit sensiblement égale à la valeur du bruit des mesures physiques associées.

En particulier, lorsque le capteur est un accéléromètre, si l'écart-type des mesures projetées sur l'axe de rotation estimé est sensiblement égal au bruit du capteur, l'axe de rotation estimé est validé comme axe de rotation sensiblement invariant du mouvement et le mouvement est en outre considéré comme planaire.

En pratique, lorsque le mobile est équipé de plusieurs capteurs, et par exemple d'au moins un magnétomètre à trois axes de sensibilité et d'un accéléromètre à trois axes de sensibilité, il est possible d'estimer l'axe de rotation selon le procédé décrit précédemment dans l'espace des mesures physiques associées à chacun des capteurs, c'est-à-dire au magnétomètre et à l'accéléromètre.

On valide ensuite comme axe de rotation sensiblement invariant du mouvement une combinaison linéaire des axes de rotation estimés pour les mesures physiques du magnétomètre et de l'accéléromètre.

Les coefficients de la combinaison linéaire sont compris non strictement entre 0 et 1 et leur somme est égale 1.

Ces coefficients sont fonction des valeurs des indicateurs des variations des axes de rotation du magnétomètre et/ou de l'accéléromètre.

Lorsque l'un des coefficients de la combinaison est égal à 0, cela revient à utiliser l'un ou l'autre capteur (magnétomètre ou accéléromètre) permettant d'obtenir l'estimation la plus fiable de l'axe de rotation sensiblement invariant.

Il est ainsi possible de s'affranchir du cas dans lequel la rotation a lieu autour de l'axe du champ physique considéré et associé au capteur, c'est-à-dire le champ de gravité pour les accéléromètres et le champ magnétique terrestre pour les magnétomètres.

En effet, dans ce cas, l'estimation de l'axe de rotation sera très mauvaise pour le capteur considéré et le procédé de détection validera automatiquement l'axe de rotation estimé selon l'autre capteur comme axe de rotation sensiblement invariant du mouvement.

A titre d'exemple, dans le cas d'un mouvement autour d'un axe vertical (ou dans un plan horizontal), comme celui d'une souris sur un bureau, l'utilisation d'un accéléromètre comme capteur inertiel dans le procédé de détection ne sera pas satisfaisant. Au contraire, l'utilisation d'un magnétomètre pourra permettre d'estimer l'axe de rotation vertical du mouvement.

On va décrire par ailleurs en référence à la figure 3, un second mode de réalisation dans lequel le procédé de détection d'un axe de rotation est mis en œuvre à partir d'une étape de calcul vectoriel réalisé sur les mesures physiques obtenues sur les trois axes de sensibilité du capteur.

Dans son principe, chaque mesure est considérée comme un vecteur dans un espace à trois dimensions.

L'axe de rotation constant dans l'espace des mesures est déterminé en réalisant l'intersection de plusieurs plans, chaque plan correspondant à l'ensemble des axes de rotation possibles permettant de passer d'un vecteur à un autre. En pratique, les vecteurs sont regroupés et analysés deux à deux afin de déterminer chaque plan.

La distance entre les plans au niveau de l'intersection est un indicateur des variations de l'axe de rotation.

Comme dans le mode de réalisation précédent, en calculant cet indicateur et en comparant la valeur de cet indicateur avec la valeur du bruit des mesures physiques du capteur, il est possible de valider l'axe de rotation estimé, c'est-à-dire l'axe correspondant à l'intersection des plans, comme axe de rotation sensiblement invariant du mouvement lorsque la valeur de l'indicateur est sensiblement égale à la valeur du bruit des mesures physiques du capteur.

En pratique, une étape d'enregistrement E31 permet d'acquérir des mesures physiques selon les trois axes de sensibilité du capteur.

On obtient ainsi N mesures selon les trois axes de sensibilité :

$$\text{mes}(t) = (m_x, m_y, m_z)(t),\ t = 0, ..., N - 1$$

Une étape de calcul E32 de tous les plans solution P(t) est ensuite mise en œuvre de la manière suivante.

On définit, pour chaque instant t, les trois vecteurs suivants :

$$\vec{v}(t) = \frac{(mes(0) + mes(t))}{\|mes(0) + mes(t)\|}\ ;\ \vec{w}(t) = \frac{(mes(0) \wedge mes(t))}{\|mes(0) \wedge mes(t)\|}\ ;\ \vec{p}(t) = \vec{v}(t) \wedge \vec{w}(t) = \begin{vmatrix} px(t) \\ py(t) \\ pz(t) \end{vmatrix}$$

En pratique, à chaque instant, l'axe de la rotation que le mobile a effectuée entre l'instant t = 0 et l'instant t appartient nécessairement au plan défini par les vecteurs $\vec{v}(t)$ et $\vec{w}(t)$, ce qui correspond encore à l'équation d'un plan $\vec{p}(t).\vec{n} = 0$.

Ainsi, s'il existe un axe de rotation constant dans le mouvement du mobile observé, alors il correspond à l'intersection de tous les plans définis par les vecteurs $\vec{v}(t)$ et $\vec{w}(t)$, avec t variant de 0 à N - 1.

A contrario, si l'intersection existe, on peut considérer que l'hypothèse selon laquelle le mouvement est réalisé autour d'un axe de rotation constant est validée et que cet axe de rotation correspond à l'intersection des plans.

En pratique, une étape de résolution E33 du système est mise en œuvre à partir de la construction d'une matrice A dont les lignes correspondent aux N échantillons et les colonnes aux trois composantes du vecteur $\vec{p}$ :

$$A = \begin{pmatrix} px(0) & py(0) & pz(0) \\ \cdots & & \cdots \\ px(N-1) & py(N-1) & pz(N-1) \end{pmatrix}$$

La recherche de l'axe de rotation $\vec{n}$ (nx, ny, nz) revient à résoudre l'équation $A\vec{n} = 0$, c'est-à-dire :

$$A\vec{n} = \begin{pmatrix} px(0) & py(0) & pz(0) \\ px(1) & py(1) & pz(1) \\ \cdots & \cdots & \cdots \\ px(N-1) & py(N-1) & pz(N-1) \end{pmatrix} \begin{pmatrix} nx \\ ny \\ nz \end{pmatrix} = \begin{pmatrix} 0 \\ 0 \\ \cdots \\ 0 \end{pmatrix}$$

Les coordonnées du vecteur $\vec{n}$ peuvent être obtenues en réalisant la décomposition en valeurs singulières de la matrice A comme décrit précédemment de telle sorte que $A = USV^T$.

De la même manière que pour l'analyse en composantes principales, les termes diagonaux sont classés par ordre décroissant.

L'axe de rotation $\vec{n}$ correspond alors à la troisième colonne de la matrice unitaire V.

Les valeurs de la diagonale de la matrice S permettent comme précédemment de calculer la valeur d d'un indicateur des variations de l'axe de rotation, qui correspond à la distance moyenne à l'hypothèse $A\vec{n} = 0$.

On observera que la résolution du système peut être facilitée lorsque le nombre d'échantillons N est important, en réalisant d'abord une triangularisation orthogonale de la matrice A et en appliquant ensuite une décomposition en valeurs singulières directement à la matrice triangularisée.

Par ailleurs, l'estimation du vecteur n peut être améliorée, notamment en supprimant de la matrice les mesures pour lesquelles mes(t) et mes(0) sont proches.

En effet, lorsque la distance entre les vecteurs est faible, l'orientation du vecteur $\vec{w}$ est fortement perturbée par le bruit des mesures, de telle sorte que l'estimation générale du vecteur $\vec{n}$ par la matrice A se trouve également perturbée.

Il est possible, afin de limiter cet effet, de supprimer de la matrice A les lignes correspondant à des observations telles que la distance entre les vecteurs mes(t) et mes(0) soit inférieure à une valeur seuil s où ce seuil s est prédéterminé en fonction du bruit des mesures obtenues en sortie du capteur associé.

On notera que ce procédé de détermination par analyse vectorielle peut être appliqué sur les mesures réalisées à partir de magnétomètres en l'absence de perturbations magnétiques ou sur des mesures obtenues en sortie d'accéléromètres en l'absence d'accélération.

En revanche, ce type de calculs vectoriels sur les mesures physiques n'est pas applicable à un vecteur inertiel de type gyromètre.

Dans les modes de réalisation décrits précédemment, il est nécessaire de disposer d'un nombre N d'échantillons au moins égal à 3 de manière à détecter l'axe de rotation.

En outre, il faut que la distance entre les mesures soit plus importante que le bruit des mesures.

Ainsi, pour un magnétomètre dont le bruit est sensiblement égal à 0,1, il est nécessaire d'avoir une distance entre les mesures d'au moins 0,3.

En pratique, cela signifie que le procédé de détection pour déterminer l'axe de rotation est particulièrement bien adapté lorsque les N échantillons permettent de couvrir des déplacements en rotation suffisamment importants autour de cet axe de rotation.

Le procédé de détection décrit précédemment peut en outre être complété par une étape de transformation des coordonnées de l'axe de rotation estimé n = (nx,ny,nz) dans le repère de mesure du capteur en coordonnées dans un repère géophysique.

Le repère géophysique peut être typiquement le repère NED (correspondant à l'acronyme « *North-East-Down* »).

A titre d'exemple, les coordonnées de l'axe de rotation dans le repère géophysique NED peuvent être calculées à partir des mesures projetées sur l'axe de rotation exprimées dans le repère du capteur de la façon suivante.

Dans le repère géophysique NED, le champ gravitationnel s'écrit g = (0,0,1) et le champ magnétique b = (bx,0,bz).

En effet, le champ magnétique est variable en fonction du lieu de mesure, les valeurs bx et bz étant la référence du champ magnétique terrestre à l'endroit où on a effectué la mesure.

8

$$bz \simeq \frac{\sqrt{3}}{2}$$

A titre d'exemple, en France, *bx*≃1/2 et .

On recherche ainsi les coordonnées nzNED, nxNED, nyNED du vecteur dans le repère géophysique NED.

Si on exprime les mesures accélérométriques dans le nouveau repère en faisant le produit matriciel newmesacc = mes$_a$V, on doit observer que la troisième composante est quasi constante. Elle reflète l'inclinaison du plan dans le repère inertiel et correspond à la coordonnée nzNED.

$$nz_{NED} = mean \ (newmesaccz)$$

De même, si on exprime les mesures accélérométriques dans le nouveau repère en faisant le produit matriciel newmesmag = Mes$_b$V, on doit observer que la troisième composante est quasi constante. Elle est le reflet conjoint de l'inclinaison du plan et de son orientation par rapport au Nord magnétique local dans le repère inertiel et correspond à la coordonnée z.

On a la relation    tmp=mean (newmesmagz)

tmp=0.5 nx$_{NED}$ + 0.86 nz$_{NED}$

ou encore    nxNED = 2*tmp-sqrt(3)*nz$_{NED}$

Enfin la coordonnée ny$_{NED}$ peut être déterminée en recherchant le vecteur normé avec les coordonnées déterminées précédemment :

$$ny_{NED} = \pm \sqrt{1 - nx_{NED}{}^2 - nz_{NED}{}^2}$$

A partir de la détection d'un axe de rotation sensiblement invariant selon le procédé décrit précédemment, il est possible d'estimer le mouvement complet d'un mobile équipé d'au moins un capteur inertiel ou magnétique à trois axes de sensibilité.

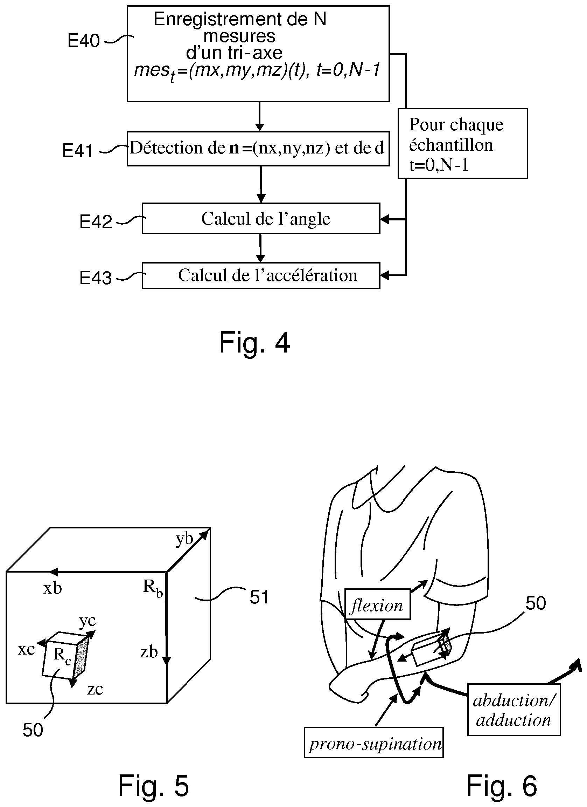

Le procédé d'estimation du mouvement d'un mobile est illustré en particulier à la figure 4.

Après une étape d'enregistrement E40 de N mesures, correspondant aux étapes d'enregistrement E10 ou E31 des figures 1 et 3, une étape de détection E41 est mise en œuvre comme décrit précédemment afin de détecter un axe de rotation représenté par ses coordonnées n = (nx,ny,nz).

Il est alors possible successivement de calculer l'angle de rotation dans une étape de calcul E42 puis l'accélération du mobile dans une étape de calcul E43, et ceci pour chaque échantillon de mesure à des instants t compris entre 0 et N - 1 et pour les échantillons suivants si l'axe de rotation ne varie plus.

En pratique, l'étape de calcul E42 de l'angle de rotation peut être mise en œuvre de la façon suivante.

Une fois l'axe de rotation estimé, on peut calculer à chaque instant l'angle θ de la rotation effectuée par un calcul analytique :

$$\theta = +2 \arcsin \frac{\left\| mes(0) - mes(t) \right\|}{\sqrt{\left\| mes(0) - mes(t) \right\|^2 + \left\| mes(0) + mes(t) \right\|^2 (\vec{w}.\vec{n})^2}}$$

Le signe de θ correspond à celui qui permet de reconstruire au mieux la mesure mes(t).

Une deuxième méthode pourrait être utilisée en estimant θ par optimisation :

$$\min_{\theta} \left( \left\| mes(t) - M(\vec{n}, \theta) mes(0) \right\|^2 \right)$$

où $M(\vec{n}, \theta)$ est la matrice de rotation d'axe n et d'angle θ.

On peut également se servir de la matrice V obtenue par analyse en composantes principales telle que décrite en référence à l'étape E14 de la figure 1, pour faire un changement de repère des données.

En reprenant les notations précédentes, les mesures dans le nouveau repère sont données par M'=VM. Dans ce repère la troisième composante sera constante.

Pour chaque instant temporel (ligne de la matrice M' de dimensions N lignes et 3 colonnes), l'angle de la rotation est l'angle formé par les 2 vecteurs 2D (leurs coordonnées correspondant aux deux premières colonnes).

En pratique, on cherche l'angle entre deux vecteurs correspondant à deux lignes de la matrice qui correspond à la rotation effectuée entre ces deux instants.

Pour cela, on peut par exemple exprimer ces vecteurs en coordonnées polaires (longueur, orientation) puis faire la différence de leurs orientations. On peut également utiliser leur produit scalaire.

Dans le cas d'un mouvement accéléré, une fois l'orientation du mobile estimée à chaque instant t à l'aide d'un magnétomètre ou d'un goniomètre, on peut estimer l'accélération du mobile à l'aide d'un accéléromètre.

Sachant que les mesures des accéléromètres s'écrivent :

$$\text{mes}(t)=M(n,\theta)(g-a)= M(n,\theta)(\text{mes}(0)-a)$$

où $M(n,\theta)$ est la matrice de rotation permettant de passer du repère fixe au repère mobile,

$\theta$ l'angle de la rotation,

a l'accélération recherchée, et

g le champ gravitationnel égal à mes(0) ds le repère de référence pris à l'instant 0.

On peut estimer l'accélération a du mobile dans le repère fixe par :

$\vec{a} = -M(\vec{n},\theta)^T mes(t)+mes(0)$ où a et mes(0) sont exprimés dans le même repère fixe.

Dans le cas particulier d'un mouvement planaire, l'accélération a doit être contenue dans le plan orthogonal à n. Le problème à résoudre revient alors à une optimisation avec contraintes :

$$\min_{\tilde{a}} \left( \left\| mes(t) - M(\vec{n},\theta)(mes(0) - \vec{\tilde{a}}) \right\|^2 \right) \quad avec \quad \vec{n} \cdot \vec{\tilde{a}} = 0$$

C'est un problème classique de moindres carrés avec contraintes linéaires que l'on peut résoudre de nouveau par des techniques de factorisation orthogonale.

Soit H la matrice de rotation telle que l'axe x devient l'axe n (ce qui s'écrit Hn=[100]'). Pour trouver H, on peut réaliser la décomposition orthogonale triangulaire de n. Soit $\tilde{H} = H(:,2 : 3)$ la matrice constituée des deux dernières colonnes de H.

Alors, on obtient l'accélération du mobile par :

$$\vec{\tilde{a}}_t = \tilde{H}\tilde{H}^T(-M(\vec{n},\theta)mes(t) + mes(0))$$

A titre d'exemple non limitatif, le mouvement observé peut-être la marche. On place par exemple le capteur sur le plateau tibial. L'alignement précis du capteur n'est plus nécessaire si on s'intéresse uniquement à l'angle décrit dans le plan sagittal de marche.

On peut appliquer la méthode d'estimation de mouvement décrite précédemment sur chaque segment temporel correspondant à une foulée ou un pas.

Par ailleurs, grâce au procédé de détection d'un axe invariant décrit précédemment, il est possible par la mise en œuvre de ce procédé, de réaliser la calibration d'un système de capteurs, c'est-à-dire de déterminer une matrice de rotation adaptée à transformer un repère de mesures du capteur en un repère prédéterminé solidaire d'un mobile portant le capteur.

Comme illustré à la figure 5, lorsque le capteur 50 est intégré à un boîtier 51, les axes xb, yb, zb du repère prédéterminé Rb sont définis par des arêtes du boîtier 51.

Le procédé de calibration qui va être décrit ci-après permet de déterminer la matrice de rotation permettant de passer du repère Rc associé au capteur 50 au repère prédéterminé solidaire du boîtier Rb.

De même, comme illustré à la figure 6, le capteur 50 peut être positionné sur une partie anatomique, et par exemple l'avant-bras d'un individu.

Le repère prédéterminé correspond alors à un repère anatomique défini par trois axes de rotation anatomique de cette partie anatomique.

Comme bien illustré à la figure 6, les axes du repère anatomique peuvent être définis par les axes de rotation respectivement d'un mouvement de flexion de l'avant-bras, d'un mouvement de prono-supination de l'avant-bras et d'un mouvement d'abduction/adduction de l'avant-bras.

Dans son principe, le procédé de calibration consiste à réaliser successivement des rotations selon les trois axes du repère prédéterminé Rb.

En cherchant ensuite pour chacun des trois mouvements, les coordonnées de l'axe de rotation correspondant dans le repère du capteur Rc, il est possible d'élaborer une matrice de rotation dans laquelle les coordonnées de chacun des vecteurs de rotation trouvés correspond à une colonne de la matrice de rotation qui permet de passer du repère de capteur Rc au repère prédéterminé solidaire du mobile Rb.

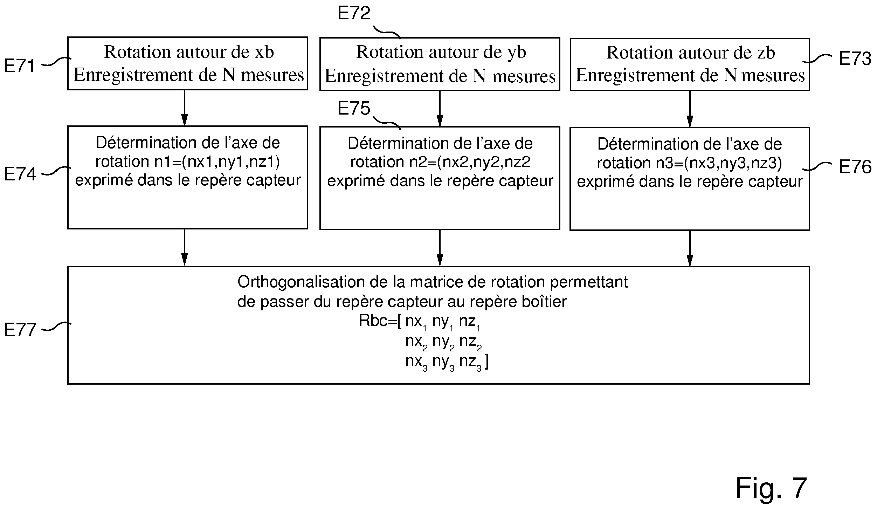

Comme illustré à la figure 7, le procédé de calibration comporte notamment des étapes d'acquisition E71, E72, E73 de mesures physiques par le capteur, correspondant respectivement à chaque rotation autour des axes xb, yb, zb du repère Rb.

Pour chaque mouvement de rotation, N échantillons de mesures sont enregistrés comme décrit précédemment en référence par exemple à l'étape d'enregistrement E10 décrite à la figure 1.

A partir de chaque enregistrement de N mesures, une étape de détermination E74, E75, E76 est mise en œuvre afin de déterminer les coordonnées des trois axes de rotation n1, n2, n3 correspondant aux axes du repère prédéterminé Rb dans le repère des mesures physiques Rc du capteur. Ces étapes de détermination de l'axe de rotation E74, E75, E76 sont mises en œuvre comme décrit précédemment par le procédé de détection d'un axe de rotation sensiblement invariant.

Une étape d'élaboration E77 d'une matrice Rbc est ensuite mise en œuvre à partir des coordonnées des axes de rotation déterminées dans le repère des mesures physiques Rc :

$$Rbc=[\quad nx1\ ny1\ nz1$$
$$nx2\ ny2\ nz2$$
$$nx3\ ny3\ nz3]$$

Lors de l'étape d'élaboration E77 de la matrice Rbc, il est également possible d'orthogonaliser la matrice ainsi formée par les trois vecteurs n1, n2, n3 de manière à la rendre unitaire. La matrice Rbc correspond alors à une matrice de rotation permettant d'exprimer les mesures du repère capteur Rc dans le repère prédéterminé Rb solidaire du mobile.

Ainsi, les mesures dans le nouveau repère Rb pourront être exprimées de la manière suivante :

$$mes_b = Rbc\ mes_c$$

dans lequel $mes_b$ correspond aux mesures dans le repère associé au boîtier et $mes_c$ correspond aux mesures dans le repère associé au capteur.

On notera que dans le procédé décrit précédemment, seules deux des trois rotations autour des axes du repère prédéterminé Rb peuvent être réalisées, les coordonnées du troisième axe de rotation étant alors obtenues en orhogonalisant la matrice formée par les cordonnées des deux premiers axes de rotation déterminés par le procédé de détection décrit précédemment.

D'autre part, le repère initial peut ne pas être orthogonal (les axes ne sont pas orthogonaux les uns par rapport aux autres). Si le repère final est orthogonal, alors la matrice Rbc qui fait le changement de repère permet donc de passer des données exprimées dans un repère non orthogonal à des données exprimées dans un repère orthogonal. Il faut obligatoirement pour cela réaliser les trois rotations et ne pas orthogonaliser la matrice Rbc obtenue.

On peut aussi choisir de passer par un repère orthogonal intermédiaire, puis de faire un changement de repère entre deux repères orthogonaux (et dans ce cas, seulement deux rotations sur les trois sont nécessaires).

Ainsi, grâce au procédé de détection d'un axe de rotation sensiblement constant décrit précédemment, il est possible de diminuer le nombre de degrés de liberté dans le mouvement d'un mobile observé et ainsi de faciliter la résolution des systèmes permettant de déterminer les paramètres de ce mouvement.

Ce procédé permet d'utiliser une centrale inertielle comprenant moins de capteurs inertiels ou magnétiques que dans les procédés de l'art antérieur et en particulier sans gyromètre.

Le procédé d'estimation d'un mouvement mettant en œuvre la détection d'un axe de rotation constant est particulièrement bien adapté au cas dans lequel l'axe de rotation est lentement variable sous réserve d'enregistrer les échantillons N de mesure sur une fenêtre temporelle de petite taille. On peut ainsi réaliser, sur chaque fenêtre temporelle, la détermination d'un axe de rotation sensiblement invariant sur cette fenêtre temporelle dès lors qu'il existe des mesures suffisamment éloignées les unes des autres sur cette fenêtre temporelle, c'est-à-dire ayant une distance suffisante par rapport au bruit des mesures du capteur.

Ainsi, pour chaque fenêtre temporelle de petite taille, on calcule la plus grande distance séparant les vecteurs de mesure pris deux à deux.

Si cette distance est inférieure à un seuil prédéterminé qui dépend du bruit des capteurs, alors on agrandit la fenêtre temporelle et on réenregistre un échantillon de N mesures sur cette nouvelle fenêtre.

Sinon, si la distance maximale entre les vecteurs de mesure est supérieure au bruit des capteurs, on calcule l'axe de rotation comme indiqué précédemment en référence au procédé de détection d'un axe de rotation sensiblement invariant et l'indicateur de variations de cet axe de rotation afin de valider l'existence d'un axe de rotation constant dans le mouvement observé.

Eventuellement, l'angle et l'accélération du mouvement sur cette fenêtre temporelle peuvent également être estimés dès lors que l'on souhaite observer le mouvement d'un mobile dans le temps.

L'ensemble des opérations est ensuite réitéré sur une fenêtre temporelle décalée dans le temps.

**Revendications**

1. Procédé de calibration d'un capteur inertiel ou magnétique à trois axes de sensibilité (X, Y, Z), ledit capteur étant un accéléromètre, un magnétomètre ou un gyromètre, pour déterminer une matrice de rotation (Rbc) adaptée à transformer les coordonnées exprimées dans un repère de mesure (Rc) dudit capteur en des coordonnées exprimées dans un repère prédéterminé (Rb) solidaire d'un mobile portant ledit capteur, **caractérisé en ce qu'**il comprend les étapes suivantes :

   - acquisition (E71, E72, E73) par ledit capteur d'au moins deux séries (E74, E75, E76) de mesures physiques (mx, my, mz) correspondant respectivement à la rotation dudit mobile selon au moins deux des trois axes (xb, yb, zb) dudit repère prédéterminé (Rb) solidaire du mobile, chacune desdites séries de mesures physiques (mx, my, mz) comprenant au moins trois échantillons (N) à des instants différents selon les trois axes de sensibilité (X, Y, Z) dudit capteur ;
   - détermination pour chacune des deux séries (E74, E75, E76) des coordonnées (nx1, nx2, nx3, ny1, ny2, ny3, nz1, nz2, nz3) de chacun des axes de rotation correspondant à l'un desdits deux des trois axes (xb, yb, zb) du repère prédéterminé (Rb) solidaire du mobile dans le repère desdites mesures physiques du capteur (Rc), la détermination des coordonnées (nx1, nx2, nx3, ny1, ny2, ny3, nz1, nz2, nz3) comprenant pour chaque série une étape d'estimation (E11-E15; E32-E34) d'un axe de rotation sensiblement invariant dans l'espace desdites mesures physiques à partir desdits au moins trois échantillons acquis (N), soit en considérant que l'axe de rotation sensiblement invariant correspond à l'axe pour lequel l'écart type des valeurs desdites mesures physiques projetées sur ledit axe est minimisé pour un magnétomètre ou un accéléromètre ou est maximisé pour un gyromètre, soit, lorsque le capteur est un magnétomètre ou un accéléromètre par un calcul vectoriel (E32, E33) sur lesdites mesures physiques ; et
   - élaboration (E77) de la matrice de rotation (Rbc) à partir desdites coordonnées (nx1, nx2, nx3, ny1, ny2, ny3, nz1, nz2, nz3) déterminées desdits au moins deux des trois axes de rotation.

2. Procédé de calibration conforme à la revendication 1, dans lequel l'étape d'estimation où l'axe de rotation sensiblement invariant correspond à l'axe pour lequel l'écart type des valeurs desdites mesures physiques projetées sur ledit axe est minimisé pour un magnétomètre ou un accéléromètre ou est maximisé pour un gyromètre comprend une étape de calcul (E14) par analyse en composantes principales, la composante principale correspondant à la valeur propre la plus faible pour le magnétomètre et l'accéléromètre et celle correspondant à la valeur propre la plus forte pour le gyromètre correspondant aux coordonnées dudit axe de rotation sensiblement invariant dans l'espace desdites mesures physiques.

3. Procédé selon la revendication 2, dans lequel l'analyse par composantes principales comporte :

   - la constitution d'une matrice (M) de telle sorte que chaque ligne correspond à un échantillon temporel des mesures et que chaque colonne correspond à une composante obtenue selon les axes de sensibilité (X, Y, Z) du capteur,
   - le calcul (E12) simultané de la moyenne obtenue pour chaque composante selon les axes de sensibilité (X, Y, Z), c'est-à-dire sur chaque colonne de la matrice (M),
   - l'obtention d'une matrice homogénéisée (Mc) par soustraction des valeurs moyennes calculées pour chaque composante de chaque élément correspondant de la matrice (M) ;
   - la réalisation d'une analyse en composantes principales (E14) sur la matrice homogénéisée (Mc).

4. Procédé selon la revendication 3, dans lequel l'analyse en composante principale sur la matrice homogénéisée (Mc) revient à chercher :

- une matrice unitaire (V) de dimension 3x3, dans laquelle chaque vecteur colonne est de norme unitaire et orthogonal aux autres vecteurs colonnes,
- une matrice diagonale ordonnées SIGMA de taille 3x3 avec des éléments positifs de la diagonale en ordre décroissant, et
- une matrice unitaire U de dimensions NxN, N correspondant au nombre d'échantillons acquis, telle que :

$$Mc = U\ SIGMA\ V^T.$$

**5.** Procédé de calibration conforme à la revendication 1, dans lequel le capteur est un accéléromètre ou un magnétomètre et l'axe de rotation sensiblement invariant correspond à l'axe pour lequel l'écart type des valeurs desdites mesures physiques projetées sur ledit axe est minimisé, **caractérisé en ce que** ladite étape d'estimation comprend une étape de calcul des moindres carrés appliquée à une combinaison linéaire desdites mesures physiques.

**6.** Procédé de calibration conforme à l'une des revendication 1 à 5, dans lequel le capteur est un accéléromètre ou un magnétomètre et l'axe de rotation sensiblement invariant correspond à l'axe pour lequel l'écart type des valeurs desdites mesures physiques projetées sur ledit axe est minimisé **caractérisé en ce que** si ledit écart type est sensiblement égal à l'écart type du bruit dudit capteur, l'axe de rotation estimé est validé comme axe de rotation sensiblement invariant dudit mouvement.

**7.** Procédé de calibration selon la revendication 1, dans lequel est mis en œuvre un calcul vectoriel (E32, E33) sur lesdites mesures physiques, dans lequel chaque mesure étant considérée comme un vecteur dans un espace à trois dimensions, les vecteurs sont regroupés deux à deux et l'axe de rotation constant dans l'espace des mesures est déterminé en réalisant l'intersection de plusieurs plans, chaque plan correspondant à l'ensemble des axes de rotations possible permettant de passer d'un vecteur à un autre.

**8.** Procédé de calibration conforme à l'une des revendications 1 à 7, **caractérisé en ce qu'**il comporte, pour l'étape d'acquisition, la réalisation successive de rotations du mobile autour d'au moins deux des trois axes du repère prédéterminé (Rb).

**9.** Procédé de calibration selon la revendication 8, dans lequel, si les rotations réalisées le sont seulement autour d'un premier axe et d'un deuxième axe des trois axes de rotations du repère prédéterminé (Rb), les coordonnées d'un troisième axe sont obtenues en orthogonalisant une matrice formée par les coordonnées desdits premier et deuxième axes de rotation déterminés.

**10.** Procédé de calibration conforme à l'une des revendications 1 à 9 ledit capteur (50) étant intégré à un boîtier (51), **caractérisé en ce que** les axes (xb,yb,zb) dudit repère prédéterminé (Rb) sont définis par les arêtes dudit boitier (51).

**11.** Procédé de calibration conforme à l'une des revendications 1 à 9, ledit capteur (50) étant positionné sur une partie anatomique, **caractérisé en ce que** ledit repère prédéterminé (Rb) correspond à un repère anatomique défini par trois axes de rotation anatomique de ladite partie anatomique.

**12.** Procédé de calibration conforme à l'une des revendications 1 à 11, **caractérisé en ce qu'**il comprend en outre les étapes suivantes :

- calcul (E16; E35) d'un indicateur (d) des variations de l'axe de rotation estimé ;
- comparaison (E16; E35) de la valeur dudit indicateur avec une valeur de seuil prédéterminée ; et
- validation de l'axe de rotation estimé comme axe de rotation sensiblement invariant dudit mouvement lorsque la valeur dudit indicateur (d) est inférieure à la valeur de seuil prédéterminée, la valeur de seuil étant avantageusement égale à la valeur du bruit des mesures physiques dudit capteur.

**Patentansprüche**

**1.** Verfahren zum Kalibrieren eines Trägheits- oder Magnetsensors mit drei Empfindlichkeitsachsen (X, Y, Z), wobei der Sensor ein Beschleunigungsmesser, ein Magnetometer oder ein Gyrometer ist, um eine Rotationsmatrix (Rbc) zu bestimmen, die dazu geeignet ist, die in einem Messkoordinatensystem (Rc) des Sensors ausgedrückten Koordinaten in Koordinaten zu transformieren, die in einem vorbestimmten Koordinatensystem (Rb) ausgedrückt werden, das mit einem den Sensor tragenden beweglichen Körper fest verbunden ist,
**dadurch gekennzeichnet, dass** es die folgenden Schritte umfasst:

- Erfassen (E71, E72, E73) von zumindest zwei Reihen (E74, E75, E76) physikalischer Messungen (mx, my, mz) durch den Sensor, die jeweils der Drehung des beweglichen Körpers um zumindest zwei der drei Achsen (xb, yb, zb) des fest mit dem beweglichen Körper verbundenen vorbestimmten Koordinatensystems (Rb) entsprechen, wobei jede der Reihen physikalischer Messungen (mx, my, mz) zumindest drei Abtastungen (N) zu unterschiedlichen Zeitpunkten entlang der drei Empfindlichkeitsachsen (X, Y, Z) des Sensors umfasst;
- für jede der beiden Reihen (E74, E75, E76) Bestimmen der Koordinaten (nx1, nx2, nx3, ny1, ny2, ny3, nz1, nz2, nz3) jeder der Rotationsachsen, die einer der beiden aus den drei Achsen (xb, yb, zb) des mit dem beweglichen Körper fest verbundenen vorbestimmten Koordinatensystems (Rb) entsprechen, in dem Koordinatensystem der physikalischen Messungen des Sensors (Rc), wobei das Bestimmen der Koordinaten (nx1, nx2, nx3, ny1, ny2, ny3, nz1, nz2, nz3) für jede Reihe einen Schritt des Schätzens (E11-E15; E32-E34) einer im Wesentlichen invarianten Rotationsachse im Raum der physikalischen Messungen ausgehend von den zumindest drei erfassten Abtastungen (N) umfasst, und zwar entweder unter der Annahme, dass die im Wesentlichen invariante Rotationsachse derjenigen Achse entspricht, für die die Standardabweichung der Werte der auf die Achse projizierten physikalischen Messungen für ein Magnetometer oder einen Beschleunigungsmesser minimiert ist bzw. für ein Gyrometer maximiert ist, oder aber, wenn der Sensor ein Magnetometer oder ein Beschleunigungsmesser ist, durch eine Vektorberechnung (E32, E33) auf den physikalischen Messungen; und
- Erstellen (E77) der Rotationsmatrix (Rbc) aus den Koordinaten (nx1, nx2, nx3, ny1, ny2, ny3, nz1, nz2, nz3), die von den zumindest zwei der drei Rotationsachsen bestimmt wurden.

2. Kalibrierungsverfahren nach Anspruch 1,
**dadurch gekennzeichnet, dass** im Schritt des Schätzens, bei dem die im Wesentlichen invariante Rotationsachse derjenigen Achse entspricht, für die die Standardabweichung der Werte der auf die Achse projizierten physikalischen Messungen für ein Magnetometer oder einen Beschleunigungsmesser minimiert bzw. für ein Gyrometer maximiert ist, der Schritt des Schätzens einen Schritt des Berechnens (E14) durch Hauptkomponentenanalyse umfasst, wobei die Hauptkomponente, die dem schwächsten Eigenwert für das Magnetometer und den Beschleunigungsmesser entspricht, und diejenige, die dem stärksten Eigenwert für das Gyrometer entspricht, den Koordinaten der im Wesentlichen invarianten Rotationsachse im Raum der physikalischen Messungen entsprechen.

3. Verfahren nach Anspruch 2,
wobei die Hauptkomponentenanalyse umfasst:

- Bilden einer Matrix (M) derart, dass jede Zeile einer zeitlichen Abtastung der Messungen entspricht und jede Spalte einer Komponente entspricht, die entlang der Empfindlichkeitsachsen (X, Y, Z) des Sensors erhalten wurde,
- gleichzeitiges Berechnen (E12) des für jede Komponente entlang der Empfindlichkeitsachsen (X, Y, Z), d. h. über jede Spalte der Matrix (M), erhaltenen Mittelwerts,
- Erhalten einer homogenisierten Matrix (Mc) durch Subtraktion der für jede Komponente berechneten Mittelwerte von jedem entsprechenden Element der Matrix (M),
- Durchführen einer Hauptkomponentenanalyse (E14) an der homogenisierten Matrix (Mc).

4. Verfahren nach Anspruch 3,
wobei die Hauptkomponentenanalyse an der homogenisierten Matrix (Mc) gleichzusetzen ist mit der Suche nach:

- einer Einheitsmatrix (V) der Größe 3x3, in der jeder Spaltenvektor eine Einheitsnorm hat und orthogonal zu den anderen Spaltenvektoren ist,
- einer geordneten Diagonalmatrix SIGMA der Größe 3x3 mit positiven Diagonalelementen in absteigender Reihenfolge, und
- einer Einheitsmatrix U der Größe NxN, wobei N der Anzahl der erfassten Abtastungen entspricht, so dass:

$$Mc = U \, SIGMA \, V^T.$$

5. Kalibrierungsverfahren nach Anspruch 1,

wobei der Sensor ein Beschleunigungsmesser oder ein Magnetometer ist und die im Wesentlichen invariante Rotationsachse derjenigen Achse entspricht, für die die Standardabweichung der Werte der auf die Achse projizierten physikalischen Messungen minimiert ist, **dadurch gekennzeichnet, dass** der Schritt des Schätzens einen Schritt des Berechnens der kleinsten Quadrate umfasst, der auf eine Linearkombination der physikalischen Messungen angewendet wird.

6. Kalibrierungsverfahren nach einem der Ansprüche 1 bis 5,

wobei der Sensor ein Beschleunigungsmesser oder ein Magnetometer ist und die im Wesentlichen invariante Rotationsachse derjenigen Achse entspricht, für die die Standardabweichung der Werte der auf die Achse projizierten physikalischen Messungen minimiert ist, **dadurch gekennzeichnet, dass** dann, wenn die Standardabweichung im Wesentlichen gleich der Standardabweichung des Rauschens des Sensors ist, die geschätzte Rotationsachse als im Wesentlichen invariante Rotationsachse der Bewegung validiert wird.

7. Kalibrierungsverfahren nach Anspruch 1, wobei eine Vektorberechnung (E32, E33) an den physikalischen Messungen erfolgt, bei der jede Messung als Vektor in einem dreidimensionalen Raum betrachtet wird, die Vektoren paarweise zusammengefasst werden und die konstante Rotationsachse im Raum der Messungen bestimmt wird, indem der Schnittpunkt mehrerer Ebenen erstellt wird, wobei jede Ebene der Gesamtheit der möglichen Rotationsachsen entspricht, die es ermöglichen, von einem Vektor zu einem anderen zu gelangen.

8. Kalibrierungsverfahren nach einem der Ansprüche 1 bis 7, **dadurch gekennzeichnet, dass** es für den Schritt des Erfassens die sukzessive Durchführung von Drehungen des beweglichen Körpers um zumindest zwei der drei Achsen des vorbestimmten Koordinatensystems (Rb) umfasst.

9. Kalibrierungsverfahren nach Anspruch 8, wobei dann, wenn die durchgeführten Drehungen nur um eine erste Achse und eine zweite Achse der drei Rotationsachsen des vorbestimmten Koordinatensystems (Rb) erfolgen, die Koordinaten einer dritten Achse durch Orthogonalisieren einer aus den Koordinaten der bestimmten ersten und zweiten Rotationsachse gebildeten Matrix erhalten werden.

10. Kalibrierungsverfahren nach einem der Ansprüche 1 bis 9,

wobei der Sensor (50) in ein Gehäuse (51) integriert ist, **dadurch gekennzeichnet, dass** die Achsen (xb, yb, zb) des vorbestimmten Koordinatensystems (Rb) durch die Kanten des Gehäuses (51) definiert sind.

11. Kalibrierungsverfahren nach einem der Ansprüche 1 bis 9, wobei der Sensor (50) auf einem anatomischen Teil positioniert ist, **dadurch gekennzeichnet, dass** das vorbestimmte Koordinatensystem (Rb) einem anatomischen Koordinatensystem entspricht, das durch drei anatomische Rotationsachsen des anatomischen Teils definiert ist.

12. Kalibrierungsverfahren nach einem der Ansprüche 1 bis 11, **dadurch gekennzeichnet, dass** es ferner die folgenden Schritte umfasst:

- Berechnen (E16; E35) eines Indikators (d) der Änderungen der geschätzten Rotationsachse;
- Vergleichen (E16; E35) des Werts des Indikators mit einem vorbestimmten Schwellenwert; und
- Validieren der geschätzten Rotationsachse als im Wesentlichen invariante Rotationsachse der Bewegung, wenn der Wert des Indikators (d) kleiner als der vorbestimmte Schwellenwert ist, wobei der Schwellenwert vorteilhafterweise gleich dem Rauschwert der physikalischen Messungen des Sensors ist.

**Claims**

1. A method for calibrating an inertial or magnetic sensor with three sensitive axes (X, Y, Z), said sensor being an accelerometer, a magnetometer or a gyrometer, for determining a rotation matrix (Rbc) suitable for transforming coordinates expressed in a measurement frame of reference (Rc) of said sensor into coordinates expressed in a predetermined frame of reference (Rb) stationary with respect to a mobile body carrying said sensor, **characterized**

**in that** it comprises the following steps:

- acquiring (E71, E72, E73) by said sensor at least two series (E74, E75, E76) of physical measurements (mx, my, mz) respectively corresponding to the rotation of said mobile body according to at least two of the three axes (xb, yb, zb) of said predetermined frame of reference (Rb) stationary with respect to said mobile body, each of said series of physical measurements (mx, my, mz) comprising at least three samples (N) at different times according to the three sensitive axes (X, Y, Z) of said sensor;
- performing determination, for each of the two series (E74, E75, E76), of the coordinates (nx1, nx2, nx3, ny1, ny2, ny3, nz1, nz2, nz3) of each of the rotation axes corresponding to one of said two of the three axes (xb, yb, zb) of the predetermined frame of reference (Rb) stationary with respect to said mobile body in the measurement frame of reference (Rc) of said sensor, the determination of the coordinates (nx1, nx2, nx3, ny1, ny2, ny3, nz1, nz2, nz3) comprising for each series a step (E11-E15; E32-E34) of estimating a substantially invariant rotation axis in the space of said physical measurements based on said at least three acquired samples (N), either by considering that the substantially invariant rotation axis corresponds to the axis for which the standard deviation of the values of said physical measurements projected onto said axis is minimized for a magnometer or an accelerometer or is maximized for a gyrometer, or, when the sensor is a magnetometer or an accelerometer, by performing a vector calculation (E32, E33) on said physical measurements; and
- producing (E77) the rotation matrix (Rbc) based on said determined coordinates (nx1, nx2, nx3, ny1, ny2, ny3, nz1, nz2, nz3) of said at least two of the three rotation axes.

2. A method for calibrating according to claim 1, in which the step of estimating in which the substantially invariant rotation axis corresponds to the axis for which the standard deviation of the values of said physical measurements projected onto said axis is minimized for a magnetometer or an accelerometer or is maximized for a gyrometer comprises a calculating step (E14) through principal component analysis, the principal component corresponding to the smallest Eigen value for the magnetometer and the accelerometer and that corresponding to the highest Eigen value for the gyrometer corresponding to the coordinates of said substantially invariant rotation axis in the space of said physical measurements.

3. A method according to claim 2, in which the principal component analysis comprises:

- constituting a matrix (M) such that each row corresponds to a temporal sample of the measurements and that each column corresponds to a component obtained according to the sensitive axes (X, Y, Z) of the sensor,
- simultaneously calculating (E12) the mean obtained for each component according to the sensitive axes (X, Y, Z), that is to say on each column of the matrix (M),
- obtaining a homogenized matrix (Mc) by subtracting the mean values calculated for each component of each element corresponding to the matrix (M);
- performing a principal component analysis (E14) on the homogenized matrix (Mc).

4. A method according to claim 3, in which the principal component analysis on the homogenized matrix (Mc) amounts to seeking:

- a unit matrix (V) of dimension 3x3, in which each column vector is of unit norm and orthogonal to the other column vectors,
- a diagonal ordered matrix SIGMA of size 3x3 with positive elements of the diagonal in decreasing order, and
- a unit matrix U of dimensions NxN, N corresponding to the number of samples acquired, such that $Mc = U \text{ SIGMA } V^T$.

5. A method for calibrating according to claim 1, in which the sensor is an accelerometer or a magnetometer and the substantially invariant rotation axis corresponds to the axis for which the standard deviation of the values of said physical measurements projected onto said axis is minimized, **characterized in that** said estimating step comprises a step of calculating the least squares error applied to a linear combination of said physical measurements.

6. A method for calibrating according to one of claims 1 to 5, in which the sensor is an accelerometer or a magnetometer and the substantially invariant rotation axis corresponds to the axis for which the standard deviation of the values of said physical measurements projected onto said axis is minimized, **characterized in that** if said standard deviation is substantially equal to the standard deviation of the noise of said sensor, the estimated rotation axis is validated as substantially invariant rotation axis of said movement.

7. A method for calibrating according to claim 1, in which a vector calculation (E32, E33) is implemented on said physical measurements, in which each measurement being considered as a vector in a three-dimensional space, the vectors are grouped together pairwise and the invariant rotational axis in the measurements space is determined by performing the intersection of several planes, each plane corresponding to the set of possible rotation axes making it possible to pass from one vector to another.

8. A calibrating method according to one of claims 1 to 7, **characterized in that** it comprises, for the acquiring step, performing successive rotations of the mobile body around at least two of the three axes of the predetermined frame of reference (Rb).

9. A method for calibrating according to claim 8, in which, if the rotations performed are only performed around a first axis and a second axis of the three rotation axes of the predetermined frame of reference (Rb), the coordinates of a third axis are obtained by orthogonalizing a matrix formed by the coordinates of said determined first and second rotation axes.

10. A method for calibrating according to one of claims 1 to 9 said sensor (50) being incorporated into a casing (51), **characterized in that** the axes (xb, yb, zb) of said predetermined frame of reference (Rb) are defined by the edges of said casing (51).

11. A method for calibrating according to one of claims 1 to 9, said sensor (50) being positioned on an anatomical feature, **characterized in that** said predetermined frame of reference (Rb) corresponds to an anatomical frame of reference defined by three anatomical rotation axes of said anatomical feature.

12. A calibrating method according to one of claims 1 to 11, **characterized in that** it comprises the following steps:

- calculating (E16; E35) an indicator (d) of the variations of the estimated rotation axis;
- comparing (E16; E35) the value of said indicator with a predetermined threshold value; and
- validating the estimated rotation axis as substantially invariant rotation axis for said movement when the value of said indicator (d) is less than the predetermined threshold value, said threshold value being advantageously equal to the value of the noise of the physical measurements of said sensor.

Enregistrement de N mesures
d'un tri-axe (mx,my,mz)(t), t=0,N-1 ⟍ E10

Constitution de la matrice M
N lignes, 3 colonnes
M=[mx(0) my(0) mz(0)
mx(1) my(1) mz(1)
...
mx(N) my(N) mz(N)]
E11

E12

Calcul de la moyenne pour
chaque composante
Mean(mx),Mean(my),Mean(mz)

Retrait de la moyenne
Mc=[mx(0)-Mean(mx) my(0) )-Mean(my) mz(0) )-Mean(mz)
mx(1)-Mean(mx) my(1) )-Mean(my) mz(1) )-Mean(mz)
...
mx(N)-Mean(mx) my(N) )-Mean(my) mz(N) )- Mean(mz)]

E13

Analyse en composantes principales
$Mc = U \ SIGMA \ V^T$
avec SIGMA ordonnée
E14

$3^{\text{ème}}$ colonne de V = axe de rotation exprimé dans
le repère capteur = (nx,ny,nz)
E15

Valeur de LAMBDA la plus
faible = $3^{\text{ème}}$ terme diagonal de
LAMBDA = indicateur de confiance
E16

Fig. 1

Fig. 2

Enregistrement de N
mesures
d'un tri-axe
$mes(t)=(mx,my,mz)(t), t=0,N-1$

E31

Calcul de tous les plans solutions

$$\vec{v}(t) = \frac{(mes(0) + mes(t))}{\left\| mes(0) + mes(t) \right\|}; \vec{w}(t) = \frac{(mes(0) \wedge mes(t))}{\left\| mes(0) \wedge mes(t) \right\|}; \vec{p}(t) = \vec{v}(t) \wedge \vec{w}(t)$$

$$p(t)=[px(t)\ py(t)\ pz(t)]'\ t=0,..N$$

E32

E33

Résolution du système :

$$A\vec{n} = \begin{pmatrix} px(0) & py(0) & pz(0) \\ px(1) & py(1) & pz(1) \\ ... & ... & ... \\ px(N-1) & py(N-1) & pz(N-1) \end{pmatrix} \begin{pmatrix} nx \\ ny \\ nz \end{pmatrix} = \begin{pmatrix} 0 \\ 0 \\ ... \\ 0 \end{pmatrix}$$

(nx,ny,nz) = axe de rotation exprimé
dans le repère capteur

E34

Indice de confiance de l'hypothèse = d
= distance moyenne à l'hypothèse
$An=0$

E35

Fig. 3

E40 — Enregistrement de N mesures d'un tri-axe $mes_t=(mx,my,mz)(t)$, $t=0,N-1$

Pour chaque échantillon $t=0,N-1$

E41 — Détection de $\mathbf{n}=(nx,ny,nz)$ et de d

E42 — Calcul de l'angle

E43 — Calcul de l'accélération

## Fig. 4

yb
xb
$R_b$
51
yc
xc
$R_c$
zb
zc
50

## Fig. 5

flexion
50
abduction/ adduction
prono-supination

## Fig. 6

E72

| E71 | Rotation autour de xb<br>Enregistrement de N mesures | Rotation autour de yb<br>Enregistrement de N mesures | Rotation autour de zb<br>Enregistrement de N mesures | E73 |

E75

| E74 | Détermination de l'axe de<br>rotation n1=(nx1,ny1,nz1)<br>exprimé dans le repère capteur | Détermination de l'axe de<br>rotation n2=(nx2,ny2,nz2<br>exprimé dans le repère capteur | Détermination de l'axe de<br>rotation n3=(nx3,ny3,nz3)<br>exprimé dans le repère capteur | E76 |

E77

Orthogonalisation de la matrice de rotation permettant
de passer du repère capteur au repère boîtier

$$Rbc=[\begin{array}{ccc} nx_1 & ny_1 & nz_1 \\ nx_2 & ny_2 & nz_2 \\ nx_3 & ny_3 & nz_3 \end{array}]$$

Fig. 7

## RÉFÉRENCES CITÉES DANS LA DESCRIPTION

**Documents brevets cités dans la description**

- FR 2838185

- US 2002103610 A

**Littérature non-brevet citée dans la description**

- **JOLLIFFE I.T.** Principal component analysis. Springer, 2002